# EUROPEAN PATENT SPECIFICATION

(11) **EP 1 220 852 B1**
(45) Date of publication and mention of the grant of the patent: **18.01.2006**
(21) Application number: 00969490.2
(22) Date of filing: 11.10.2000
(51) Int. Cl.: C07D 401/06, C07D 243/08, A61K 31/4709, A61K 31/551

(54) **SUBSTITUTED DIAZEPANS**
SUBSTITUIERTE DIAZEPAN
DIAZEPANS SUBSTITUES

(30) Priority: 13.10.1999 US 416815
(43) Date of publication of application: 10.07.2002
(73) Proprietor: Novartis AG, 4056 Basel (CH); Novartis Pharma GmbH, 1230 Wien (AT)
(72) Inventor: ALBERT, Rainer, CH-4052 Basel (CH); EHRHARDT, Claus, 79541 Lörrach (DE); HOMMEL, Ulrich, 79379 Müllheim (DE); KALLEN, Jörg, CH-4054 Basel (CH); MEINGASSNER, Josef, Gottfried, A-2380 Perchtoldsdorf (AT); ROCHE, Didier, F-6008 Lyon (FR); WATTANASIN, Sompong, Hopatcong, NJ 07843 (US); WEITZ-SCHMIDT, Gabriele, 79189 Bad Krozingen (DE); WELZENBACH, Karl, CH-4056 Basel (CH)
(74) Representative: Gros, Florent
(86) International application number: PCT/EP2000/010025
(87) International publication number: WO 2001/027102

(56) References cited:
- EP-A- 0 365 992
- EP-A- 0 519 433
- WO-A-99/20618
- LAMMEK B ET AL: "BIOLOGICALLY ACTIVE ANALOGUES OF ARGININE VASOPRESSIN CONTAINING CONFORMATIONALLY RESTRICTED DIPEPTIDE FRAGMENTS" JOURNAL OF PEPTIDE RESEARCH,DK,MUNKSGAARD INTERNATIONAL PUBLISHERS, COPENHAGEN, vol. 51, no. 2, 1 February 1998 (1998-02-01), pages 149-154, XP000730043 ISSN: 1397-002X
- T YAMASHITA ET AL: "Synthesis and opiate activity of Pseudo-tetrapeptides" CHEM PHARM BULL, vol. 45, no. 12, 1997, pages 1940-1944, XP000941771
- M PIERCY: "Analgesic Activities of Spinal Cord Substance P Antagonists" BRAIN RESEARCH, vol. 385, no. 1, 1986, pages 74-85, XP000945073

## Description

The present invention rentes to diazepanes, a process for their production, their use as a pharmaceutical and pharmaceutical preparations containing them.

More particularly the present invention provides a compound of formula I 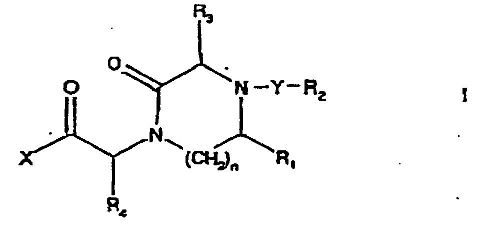
wherein
- n: is 1,2 or 3.
- R₁: is H, C₁₋₄alkyl; aryl; or aryl-C₁₋₄alkyl,
- Y: is C₁₋₄alkylene; -CO-C₁₋₄alkylene; -CO-C₂₋₅alkenylene; -CO-NH-; -CO-C₁₋₃alkylene-NH-; or -CO-O-,
- R₂: is phenyl, naphthyl. dihydro- or tetrahydro-naphthyl, biphenylyl, pyridyl, quinolyl, isoquinolyl, dihydro-, tetrahydro-quinolyl or -isoquinolyl, benzo-thienyl, indolyl or pyndyl-phenyl, each being optionally substituted by CF₃, halogen, OH, C₁₋₄alkoxy, amino, mono- or di-C₁₋₄alkyl substituted amino, phenyl, benzyl or C₁₋₄alkyl optionally substituted by amino,
- R₃: is the side chain present on the Cα of an α-amino acid,
- R₄: is biphenylyl; or benzyl, hydroxy-benzyl, α- or β-naphthyl-methyl, 5,6,7,8-tetrahydro-β-naphthyl-methyl or indolyl-methyl, each being optionally substituted on the ring by CF₃ₐ halogen, OH, C₁₋₄alkoxy, amino, mono- or di-C₁₋₄alkyl substituted amino, phenyl, benzyl or C₁₋₄alkyl optionally substituted by amino, and
- X: is -CN; -NR₅R₅; or -O-R₈ wherein R₅ is H, C₁₋₆alkyl, aryl or aryl-C₁₋₄alkyl, R₆ is H or C₁₋₆alkyl and Rₐ is H, C₁₋₄alkyl, aryl or aryl-C₁₋₄alkyl,

in free form or in salt form.

Alkyl may be linear or branched. When alkyl is substituted by amino, it is preferably monosubsthuted, more preferably terminally substituted. Aryl may be e.g. optionally substituted phenyl, naphthyl or cuhydro- or tetrahydro-naphthyl. Aryl-C₁₋₄alkyl may be e.g. phenyl-C₁₋₄alkyl, e.g. benzyl, optionally substituted on the ring. Examples of substitutents are e.g. halogen, OH, CF₃ or NH₂. Preferably aryl and aryl-C₁₋₄alkyl are unsubstituted.

Halogen may be F, Cl or Br.

When R₂ is tetrahydro-quinolyl it is e.g. 1,2,3,4-tetrahydro-quinolyl. By Optionally substituted tetrahydro-quinolyl or indolyl is also meant tetrahydroquinolyl or indolyl wherein the nitrogen is substituted, e.g. by C₁₋₄alkyl, e.g. methyl or ethyl.

The α-amino acid or aromatic α-amino add from which is derived the side chain present on the Cα as R₃, may be natural or non natural Suitable examples as Rₐ include e.g. propyl, isopropyl, butyl, isobutyl, 1-methyl-propyl, phenyl, benzyl or aminobutyl.

By optionally ring substituted indolyl as R₄ is also meant indolyl wherein the nitrogen is substituted, e.g. by C₁₋₄alkyl, e.g. methyl or ethyl, or benzyl.

The compounds of formula 1 may exist in free form or in salt form, e.g. addition salts with e.g. organic or inorganic acids, for example, hydrochloric acid, acetic acid, or salts obtainable when Ra is H as salts with a base, e.g. alkali salts such as sodium or potassium, or substituted or unsubstituted ammonium salts.

It will be appreciated that the compounds of formula I may exist in the form of optical isomes, racemates or diastereoisomers as well as in the form of cis or trans conformers. For example, the carbon atom bearing the substituent R₂ or R₄, respectively, is asymmetric and may have the D- or L-configuration. For example, the carbon atom bearing R₄ has preferably the D-configuration when R₄ is α-naphthyl-methyl; it preferably has the L-configuration when R₄ is β-naphthyl-methyl. It is to be understood that the present invention embraces all enantiomers and conformers and their mixtures. Similar considerations apply in relation to starting materials exhibiting asymmetric carbon atoms as mentioned above.

In the compounds of formula I, the following significances are preferred individually or in any sub-combination:
- 1.: n=2
- 2.: Y is -CH₂-; -CO-CH₂-; -CO-CH₂-CH₂-; -CO-NH-; -CO-CH₂-NH-; or -CO-CH=CH-;
- 3.: R₁ is C₁₋₄alkyl, preferably methyl or ethyl, more preferably methyl;
- 4.: R₁ is C₁₋₄alkyl, preferably as indicated, and the carbon atom bearing it has the R-configuration;
- 5.: R₂ is phenyl; phenyl substituted as indicated above, e.g. as indicated in the examples; naphthyl; pyridyl, preferably 2-, 3- or 4-pyridyl; pyridyl-phenyl; quinolyl, isoquinolyl, tetrahydro-quinolyl or substituted quinolyl or isoquinolyl, preferably 2-, 4-, 6- or 8-quinolyl, optionally substituted as indicated above, e.g. as in the examples below;
- 6.: R₂ is quinolyl or isoquinolyl; or substituted quinolyl or isoquinolyl, e.g. by OH, OCH₃ or phenyl; preferably quinolyl;
- 7.: R₃ is isopropyl; n-butyl; isobutyl; or phenyl;
- 8.: R₄ is α- or β-naphthyl-methyt;
- 9.: R₄ is β-naphthyl-methyl and the carbon atom bearing R₄ has the L-(S)-configuration;
- 10.: R₄ is α-naphthyl-methyl and the carbon atom bearing R₄ has the D-(R)-configuration;
- 11.: X is -NR₅R₆;
- 12.: R₅ is H; C₁₋₃alkyl; or benzyl;
- 13.: R₆ is H or CH₃, preferably H.

The present invention also includes a process for the production of a compound of formula I, which process comprises appropriately substituting a corresponding compound of formula II as defined below, e.g.:
a) for the production of a compound of formula I wherein Y is -CO-C₁₋₄alkylene or -CO-C₂₋₅alkenylene, reacting a compound of formula II 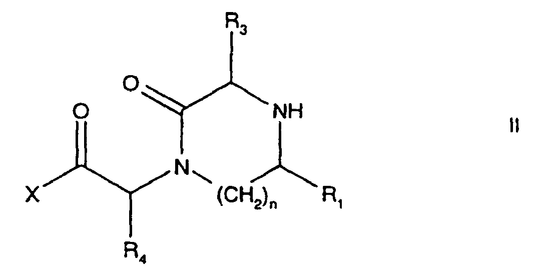
   wherein R₁, R₃, R₄, X and n are as defined above,
   with a compound of fomiula IIIa

   R₂-Y'-OH IIIa

   wherein R₂ is as defined above and Y' is -CO-C₁₋₄alkylene or -CO-C₂₋₅alkenylene or a functional derivative thereof;
b) for the production of a compound of formula I wherein Y is C₁₋₄alkylene, reacting a compound of formula II above, with a compound of formula IIIb

   R₂-Y"-CHO IIIb

   wherein R₂ is as defined above and Y" is a direct bond or C₁₋₃alkylene, under reducing conditions;
c) for the production of a compound of formula I wherein Y is -CO-NH- or -CO-C₁₋₃₋allryiene-NH,
   reacting a compound of formula 11 above with a compound of formula IIIc

   X₁ - CO - Y"' - X₂ IIIc

   wherein Y"' is -CO-NH or -CO-C₁₋₃alkylene-NH and each of X₁ and X₂ is a leaving
   group, e.g. Br, and subsequently reacting the resulting compound with R₂ - NH₂; and
d) for the production of a compound of formula I wherein Y is -CO-NH-reacting a compound of formula II with R₂-N=C=O,
   and, where required, converting the resulting compound of formula I obtained in free form to a salt form or vice versa.

A functional derivative of a compound of formula IIIa includes e.g. a halide, ester or anhydride.

Process step a) may be carried out according to known acylation methods, e.g. in liquid phase or in solid phase. The latter case may particularly be suitable for the preparation of a compound of formula I wherein X is NR₅R₆: in such a case, the compound of formula II is attached to a resin, e.g. a commercially available resin, e.g. by NH. Once acylation is complete, the desired compound of formula 1 is cleaved from the resin e.g. by acidic hydrolysis.

Process step b) may be carried out according to known methods, in the presence of a reducing agent, e.g. NaCNBH₃. Process steps c) and d) may be performed according to known methods.

The compounds of formula 11 may be produced e.g. by cyclisation of a compound of formula IV 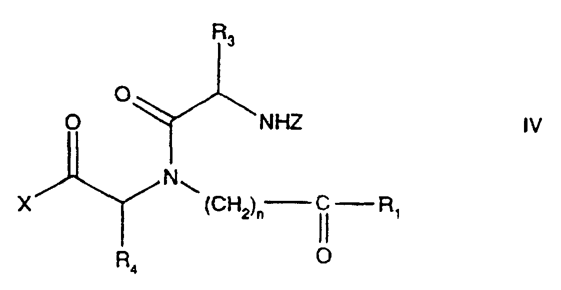
wherein R₁, R₃, R₄, X and n are as defined above and Z is an amino protecting group, in the presence of a reducing agent.

Suitable reducing agents include e.g. NaCNBH₃, Na BH(CH₃COO)₃, or Na triacetoxy borohydride. The compounds of formula 11 may be prepared in liquid or solid phase. In the latter case, the compound of formula IV is attached to a resin by an appropriate group X', e.g. NH. Suitable N-protecting groups may be e.g. as disclosed in "Protective Groups in Organic Synthesis", T.W. Greene, J. Wiley & Sons NY(1981), 219-287, e.g. alcoxycarbonyl such as methoxycarbonyl or t-butyloxycarbonyl, allyloxycarbonyl, arylmethoxycarbonyl such as Fmoc, or benzyloxycarbonyl.

Insofar the production of the starting materials is not particularly described, the compounds are known or may be prepared analogously to methods known in the art or as disclosed hereafter.

The following Examples are illustrative of the invention.
Fmoc = 9-Fluorenyl methoxy carbonyl
Boc = tert-butoxy-carbonyl
RT = room temperature
THF = tetrahydrofurane
TFA = trifluoroacetic acid
DMF = dimethylformamide
DCCI = dicyclohexylcarbodiimide
EADC = N-(3-dimethylaminopropyl)-N'-ethylcarbodiimide hydrochloride
NMM = N-methyl morpholine
DIPCI = diisopropylcarbodiirnide
HOBt = hydroxy benzotriazole
DIPEA = diisopropyl ethyl amine, also called Hünig's base
AcOH = acetic acid
Nal = naphthyl-alanine
Phg = phenyl-glycine

### Example 1: (S)-2-[(3S,5R)-3-Isobutyl-5-methyl-2-oxo-4-(quinolin-6-yl-acetyl)-[1,4]diazepan-1-yl]-3-(naphthaten-2-yl)-propionamide

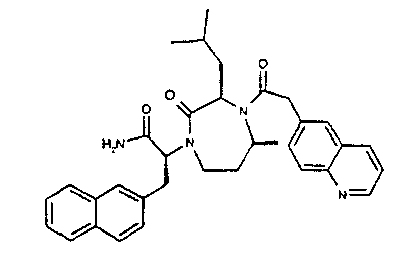

(S)-2-[(3S,5R)-3-isobutyl-5-methyl-2-oxo-[1,4]diazepan-1-yl]-3-(naphthalen-2-yl)-propionamide (382 mg, 1 mmol) is added to a solution of 6-quinolylacetic acid (372 mg, 2 mmol), Hünig's base (170 µl, 1 mmol) and EADC (383 mg, 2 mmol) in CH₂Cl₂ (50 ml). After 16 hours at RT the reaction mixture is extracted with 0.1N HCl and then with 5% NaHCO₃ solution. Pure title compound is isolated after silica gel chromatography using ethyl acetate --> ethyl acetate/MeOH (9/1) as mobile phase.
MH⁺: 551.4 (ESI)
α_{D}²⁰ = -12.5° (c= 0.12 95% AcOH)
(S)-2-[(3S,5R)-3-isobutyl-5-methyl-2-oxo-[1,4]diazepan-1-yl]-3-(naphthalen-2-yl)-propionamide, used as starting material, may be prepared as follows:

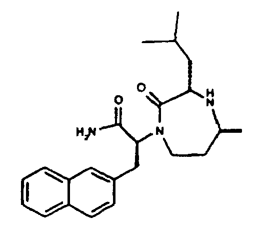

### a) N-Boc-β-L-Nal-NH₂:

DCCI (5.0 g, 24 mmol) is added to a solution of N-Boc-β-L-Nal-OH (6.3 g, 20 mmol), HOBt (3.7 g, 24 mmol) and NH₄OH (25%) (2.0 ml, 24 mmol) in DMF (100 ml) at RT. After 16 hours at RT the solution is filtered and poured into 2 liter 5% aqueous NaHCO₃ solution. The resulting precipitate is filtered off and is dissolved in ethyl acetate (500 ml). After drying over Na₂SO₄ the solvent is removed under reduced pressure. The title compound forms an amorphous solid from c-hexane.

### b) H-β-L-Nal-NH₂,HCl:

N-Boc-β-L-Nal-NH₂ (6.25 g, 20 mmol) is treated with 60 ml neat TFA at 0° C for 15 minutes. After that the clear solution is poured into 2 1 diethylether containing 100 ml of a 3N HCl/diethylether solution. The resulting precipitate is filtered off and is dried at 40 °C for 2 hours.

### c) N^{α}-(3-oxo-butyl)-β-L-Nal-NH₂:

H-β-L-Nal-NH₂.HCl (4.3 g, 17 mmol) is dissolved in 200 ml dioxane at room temperature. After addition of Hünig's base (3.4 ml; 20 mmol) and methylvinylketone (25 ml, 300 mmol) the reaction is kept at room temperature for 16 hours. The reaction is concentrated to 100 ml and the remaining solution is poured into methyl-tert.-butylether (4 1). The precipitate is filtered off, then washed and is used for the next step without further purification.

### d) N^{α}-Boc-Leu-Nα-(3-oxo-butyl)-β-L-Nal-NH₂:

N^{α}-(3-oxo-butyl)-β-L-Nal-NH₂ (4.5 g, 16 mmol) is added to a solution of Boc-Leu-OH x H₂O (14.8 g, 64 mmol), NMM (3.1 ml, 64 mmol) and DIPCI (5 ml, 64 mmol) in DMF (100 ml). The reaction is kept at RT for 16 hours, filtered and then poured into 2 liter 5% aqueous NaHCO₃. The aqueous phase is decanted and the sticky residue is dissolved in ethyl acetate and dried over Na₂SO₄. Purification is achieved by silica gel chromatography using ethyl acetate --> ethyl acetate/MeOH (9/1) as mobile phase.

### e) H-Leu-N^{α}-(3-oxo-butyl)-β-L-Nal-NH₂.HCl:

N^{α}-Boc-Leu-N^{α}-(3-oxo-butyl)-β-L-Nal-NH₂ (2.9 g, 6 mmol) is treated with 30 ml neat TFA at 0° C for 15 minutes. After that the clear solution is poured into 1 l diethylether containing 50 ml of a 3N HCl/diethylether solution. The resulting precipitate is filtered off and is dried at 40 °C for 2 hours.

### f) (S)-2-[(3S,5R)-3-isobutyl-5-methyl-2-oxo-[1,4]diazepan-1-yl]-3-(naphthalen-2-yl)-propionamide

H-Leu-N^{α}-(3-oxo-butyl)-β-L-Nal-NH₂.HCI (2.5 g, 6 mmol) is dissolved in dioxane/water (4/1, 100 ml) and the pH is adjusted to 5.4 with 1 N aqueous NaOH. The cyclization reaction is carried out with NaCNBH₃ (0.79 g, 12 mmol) at RT. After 30 minutes reaction time most of the dioxane is removed under reduced pressure and the aqueous phase is extracted 3 times with ethyl acetate. Pure endproduct is obtained after silica gel chromatography using ethyl acetate --> ethyl acetate/MeOH (9/1) as mobile phase.
MH⁺: 382.2 (ESI)
α_{D}²⁰: -46.9 (c=0.18 95% AcOH)

### Example 2: (S)-2-[(3S,5R)-3-Isobutyl-5-methyl-2-oxo-4-(quinolin-6-yl-acetyl)-[1,4]diazepan-1-yl]-N-methyl-3-(naphthalen-2-yl)-propionamide

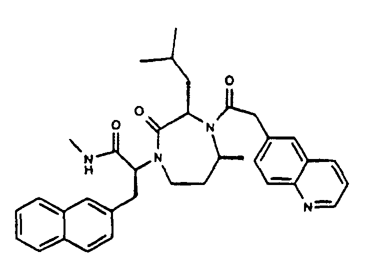

The title compound is obtained by following the procedure of Example 1 and starting with (S)-2-[(3S,5R)-3-isobutyl-5-methyl-2-oxo-[1,4]diazepan-1-yl]-N-methyl-3-(naphthalen-2-yl)-propionamide instead of (S)-2-[(3S,5R)-3-isobutyl-5-methyl-2-oxo-[1,4]diazepan-1-yl]-3-(naphthalen-2-yl)-propionamide.
MH⁺: 565.4 (ESI)
α_{D}²⁰: +19.9° (c=0.18 95% AcOH)

### (S)-2-[(3S,5R]-3-isobutyl-5-methyl-2-oxo-[1,4]diazepan-1-yl]-N-methyl-3-(naphthalen-2-yl)-propionamide

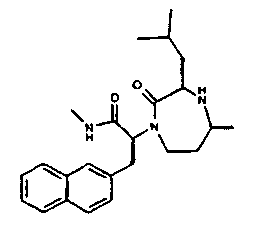

used as starting material may be prepared as disclosed in Example 1, steps (a) to (f) but employing methylamine-hydrochloride (1.4 g, 24 mmol) and NMM (2.6 ml, 24 mmol) instead of NH₄OH (25%) in step a).
MH⁺: 396.0 (ESI)
α_{D}²⁰: -67.0° (c=0.50 95% AcOH)

### Example 3: (R)-2-[(3S,5R)-5-Methyl-2-oxo-3-phenyl-4-(quinolin-6-yl-acetyl)-[1,4]diazepan-1-yl)-3-(naphthalen-1-yl)-propionamide

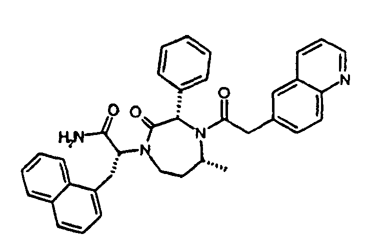

The title compound is prepared by following the procedure of Example 1 but starting from (R)-2-[(3S,5R)-5-methyl-2-oxo-3-phenyl-[1,4]-diazepan-1-yl]-3-(naphthalen-1-yl)-propionamide and using Boc-α-D-Nal-OH in step a) instead of N-Boc-β-L-Nal-OH and Boc-L-Phg-OH in step d) instead of Boc-Leu-OH.
MH⁺: 571 (ES⁺)
α_{D}²⁰: +187.6 (c=0.15 95% AcOH)

### Example 4: (S)-2-[(3S,5R)-3-Isobutyl-5-methyl-2-oxo-4-(β-naphthyl-acetyl)-[1,4]diazepan-1-yl]-3-(naphthalen-2-yl)-propionamide

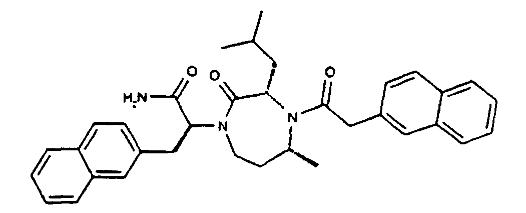

a) Commercially available Fmoc-Rink-amide MBHA resin (0.61 mmol/g, 1 eq.) is washed with isopropanol (1 x), DMF (2 x), and then treated with a 20% solution of piperidine in DMF (4 x 5 min). The resin is then washed with DMF (1 x), isopropanol (2 x) and DMF (4 x). In a separated vessel, the Fmoc-β-L-Nal-OH (6 eq.) is activated by the sequential addition of a 0.5 M solution of HOBt (6 eq.) in DMF and a 2 M solution of DIPCI (6 eq.). After stirring for 10 min this solution is transferred to the resin. DIPEA (1 eq.) is added to the suspension of resin which is shaken over night at RT. The shaking vessel is then drained and the resin is washed with DMF (4 x), EtOH (2 x), CH₂Cl₂ (3 x), EtOH (2 x), CH₂Cl₂ (3 x) and dried over a N₂ stream. The resulting resin gives a negative result (yellow color) with the bromophenol blue test and the loading of the resin is controlled by Fmoc titration after treatment of 2-5 mg of resin with 20% piperidine in DMF.
b) The resin is washed with isopropanol (1 x), DMF (2 x), and then treated with a 20% solution of piperidine in DMF (4 x 5 min). The resin is then washed with DMF (1 x), isopropanol (2 x) and DMF (4 x). A 0.5 M solution of methyl vinylketone in DMF is then added and the resulting suspension is shaken over night at RT. The shaking vessel is then drained and the resin is washed with DMF (4 x), CH₂Cl₂ (3 x), DMF (2 x), CH₂Cl₂ (2 x) and DMF (3 x). The resulting resin gives a positive result (deep blue color) with the bromophenol blue test and is used directly in the next step without drying.
c) The resin is washed with DMF (2 x). A 0.5 M solution of Fmoc-Leu-OH (10 eq.) in DMF is then added followed by DIPCI (5 eq.). The resulting suspension is shaken for 48 h at RT. The shaking vessel is drained and the resin is washed with DMF (4 x), EtOH (2 x), CH₂Cl₂ (3 x), EtOH (2 x), CH₂Cl₂ (3 x) and is dried over a N₂ stream. Under completion, the resulting resin gives a negative result (yellow color) with the bromophenol blue test. The reaction is re-runned when a positive result (deep blue color) is obtained.
d) The resin is washed with DMF (1 x) and then treated with a 20% solution of piperidine in DMF (4 x 5 min). The resin is washed with DMF (2 x), CH₂Cl₂ (2 x), DMF (4 x) and then treated with a 0.5 M solution of NaBH₃CN (10 eq.) in 1% AcOH buffered DMF. After over night shaking at RT, the vessel is drained and the resin is washed with 1% AcOH/DMF (4 x), MeOH (4 x), CH₂Cl₂ (3 x), MeOH (3 x), CH₂Cl₂ (4 x) and dried over a N₂ stream. The resulting resin gives a positive result (deep blue color) with the bromophenol blue test.
e) The resin is washed with DMF (2 x). A 0.4 M pre-mixed solution of β-naphthylacetic acid (10 eq.) and DIPCI (10 eq.) is then added followed by DIPEA (2 eq.). After over night shaking, the vessel is drained and the resin is washed with DMF (4 x), CH₂Cl₂ (3 x), DMF (2 x), CH₂Cl₂ (4 x). The resin is then treated with 50% TFA/CH₂Cl₂ and shaken at RT for 60 min. The resin is washed with 50% TFA/CH₂Cl₂ (1 x), H₂O (2 x), CH₂Cl₂ (3 x) and the filtrate is concentrated using a N₂ stream. The residue is dissolved in a small amount of ethyl acetate and then purified on a short silica gel column using ethyl acetate → ethyl acetate/methanol (1/1) as mobile phase. Fractions containing the title compound are concentrated and taken then into CH₃CN/H₂O 1/3 and freeze dried to give a white powder.

White powder; Rt 18.25 min (HPLC RP C₁₈, 0-100 % CH₃CN in H₂O/20 min); MS *m*/*z* (relative intensity) (ESI) 533 (100), 550 (73), 572 (10); Anal. Calcd for C₃₅H₃₉N₃O₃: C 76.47, H 7.15, N 7.64; Found: C 76.18, H 7.12, N 7.76.

### Example 5: (S)-2-[(3S,5R)-3-Isobutyl-5-methyl-2-oxo-4-(β-naphthyl-acetyl)-[1,4]diazepan-1-yl]-3-(naphthaten-2-yl)-propionic acid

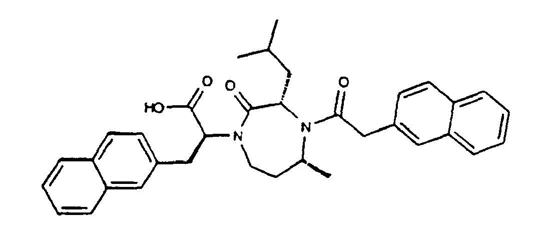

This compound is isolated during the purification on silica gel of the compound of Example 4.
The compounds of formula X 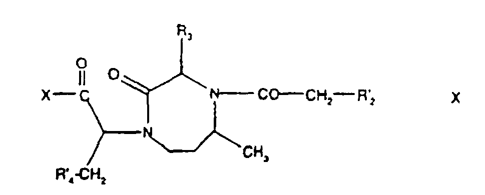
wherein R'₂, R₃, R'₄ and X are as defined in Table 1 below,
may be prepared by following the procedures of Ex. 1 and 4 and using the appropriate starting materials.

**Table 1**

| Ex | R'₂ | R₃ | R'₄ | X | MH+ (ESI) |
|---|---|---|---|---|---|
| 6 | β-naphthyl | n-butyl | β-naphthyl | NH₂ | 550.3 |
| 7 | phenyl | isobutyl | β-naphthyl | NH₂ | 500.3 |
| 8 | 4-pyridyl | isobutyl | β-naphthyl | NH₂ | 501.3 |
| 9 | 6-quinolyl | isobutyl | α-naphthyl | NH₂ | 551.3 |
| 10 | 3-quinolyl | isobutyl | β-naphthyl | NH₂ | 551.3 |
| 11 | 6-quinolyl | isobutyl | β-naphthyl | OCH₃ | 566.3 |
| 12 | 6-quinolyl | isobutyl | β-naphthyl | NH-benzyl | 641.4 |
| 13 | β-naphthyl | isobutyl | β-naphthyl | NHCH₃ | 564.3 |
| 14 | 4-Bromo-phenyl | isobutyl | β-naphthyl | NH₂ | 580.3 |
| 15 | β-naphthyl | phenyl | α-naphthyl | NH₂ | 568.3 |
| 16 | 3-indol-3-yl | isobutyl | β-naphthyl | NH₂ | 539.4 |
| 17 | N-CH₃-indol-3-yl | isobutyl | β-naphthyl | NH₂ | 553.4 |
| 18 | p-(3-pyridyl)-phenyl | isobutyl | β-naphthyl | NH₂ | 577.4 |
| 19 | 6-quinolyl | isobutyl | 5,6,7,8-tetrahydro-β-naphthyl | NH₂ | 555.4 |
| 20 | 3,4-dichloro-phenyl | phenyl | α-naphthyl | NH₂ | 488.0 |
| 21 | 6-quinolyl | isobutyl | 4-bromo-phenyl | NH₂ | 579/581 |
| 22 | p-amino-phenyl- | isobutyl | α-naphthyl | NH₂ | 551.3 |
| 23 | p-(NH₂-CH₂)-phenyl- | isobutyl | α-naphthyl | NH₂ | 529.4 |
| 24 | 6-quinolyl | isobutyl | α-naphthyl | NH₂ | 551 |
| 25 | p-[di(CH₃)-amino]-phenyl | isobutyl | α-naphthyl | NH₂ | 543 |
| 26 | p-chloro-phenyl | isobutyl | α-naphthyl | NHCH₃ | 549 |
| 27 | p-ethoxy-phenyl | isobutyl | α-naphthyl | NHCH₃ | 558 |
| 28 | 1-CK₃-1.2,3,4-tetrahydro-6-quinolyl | isobutyl | α-naphthyl | NHCH₃ | 583 |
| 29 | 3-bromo-5-pyridyl | isobutyl | α-naphthyl | NH₂ | 580 |
| 30 | 4-quinolyl | isobutyl | β-naphthyl | NHCH₃ | 564 |
| 31 | 7-CH₃O-3-quinolyl | isobutyl | β-naphthyl | NHCH₃ | 595.4 |
| 32 | 2-phenyl-6-quinolyl | isobutyl | β-naphthyl | NHCH₃ | 641 |
| 33 | 1-OH-3-isoquinolyl | isobutyl | β-naphthyl | NHCH₃ | 581 |
| 34 | 3,4-di(CH₃O)-phenyl | isobutyl | β-naphthyl | NHCH₃ | 574 |
| 35 | 3-CH₃O-4-OH-phenyl | isobutyl | β-naphthyl | NHCH₃ | 560 |
| 36 | β-naphthyl | phenyl | α-naphthyl | CN | 560.3 |

The compounds of formula XI 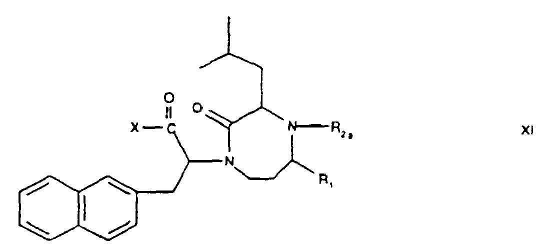
wherein R₂ₐ, R, and X are as defined in Table 2 below,
may be prepared by following the procedures of Ex 1 and 4 and using the corresponding starting materials.

**Table 2**

| Ex | R₂ₐ | R₁ | X | MH+ (ESI) |
|---|---|---|---|---|
| 37 | 4-quinolyl-CH₂-CH₂-CO- | CH₃ | NH₂ | 565.4 |
| 38 | 6-quinolyl-CH₂-CO- | CH₂CH₃ | NH₂ | 565.4 |
| 39 | 4-quinolyl-CH=CH-CO- | CH₃ | NH₂ | 563.0 |
| 40 | 4-quinolyl-CH=CH-CO- | CH₃ | NHCH₃ | 577.4 |
| 41 | α-naphthyl-CH=CH-CO- | CH₃ | NHCH₃ | 562.2 |
| 42 | 4-pyridyl-CH=CH-CO- | CH₃ | NHCH₃ | 513.0 |
| 43 | benzyl^{a} | CH₃ | NHCH₃ | 486 |
| 44 | phenyl-NHCO-^{b} | CH₃ | NHCH₃ | 515 |
| 45 | β-naphthyl-NHCO-^{b} | CH₃ | NHCH₃ | 565 |
| 46 | (p-F-phenyl)-NH-CH₂-CO- | CH₃ | NHCH₃ | 547 |

| | | | | |
|---|---|---|---|---|
| ^{a}: prepared according to process step b) | | | | |
| ^{b}: prepared according to process step c) | | | | |

The compounds of Examples 1 to 46 may also be prepared as R- or S-enantiomers or as mixtures as regards the carbon atoms bearing the substitutents R₁ (when this is other than H), R₃ and R₄, respectively.

The compounds of formula 1, in free form or in pharmaceutically acceptable salt form exhibit valuable pharmacological properties, e.g. inhibiting activity of LFA-1/ICAM-1, -ICAM-2 or -ICAM-3 interactions or inhibiting inflammation, e.g. as indicated in in vitro and in vivo tests and are therefore indicated for therapy.

### A. In vitro: Cell Free Assay

The assay measures the binding of soluble human ICAM-1 to immobilized human LFA-1. LFA-1 is purified from JY cells, a human lymphoblastoid B cell-line, by immunoaffinity chromatography as described by Dustin et a/. (J. Immunol. 148, 2654-2663, 1992). ICAM-1 mouse Cκ fusion protein (ICAM-1) is produced using the baculovirus system as described by Weitz-Schmidt *et al.* (Anal. Biochem.238,184-190, 1996).

Purified LFA-1 is diluted 1:20 in phosphate buffered saline (PBS) containing 2 mM MgCl₂, pH 7.4 and coated onto microtitre plates (Nunc) at 37°C for 3h. Plates are blocked with 1% heat-treated BSA in PBS for 2 hours at 37°C followed by a washing step using PBS, 2mM MgCl₂, 1% fetal calf serum, pH 7.4 (assay buffer). Compounds dissolved at 10 mM in DMSO are diluted in assay buffer and added to the plates. Biotinylated recombinant ICAM-1 in assay buffer (6 µg/ml) is added and allowed to bind at 37°C for one hour. After incubation, wells are washed with assay buffer. Streptavidin-peroxidase diluted 1:5000 in assay buffer is added and incubated for 45 min at 37ºC. Plates are then washed with assay buffer and 2.2'-azino-bis(3-ethylbenzothiazoiine-6 sulfonic acid) diammonium salt substrate solution is added to each well. The reaction is stopped after 20 min and bound ICAM-1 is determined by measuring the optical density at 405 nm in a microplate reader.

In this assay, compounds of formula I inhibit adhesion of LFA-1 to ICAM-1 with an IC₅₀ ≤ 30 µM, preferably 0.05 to 30 µM. Compounds of Examples 1 and 3 have an IC₅₀ of 0.44 and 0.07 µM, respectively, in this assay.

### B. In vivo

### i) Murine Thioglycollate Induced Peritonitis

Thioglycolate is injected i.p. to mice and immediately thereafter the compound to be tested is given s.c. The mice are killed after 4 hours, the peritoneal cavity lavaged and total number of neutrophils in the lavage fluid is determined.
In this assay, the compounds of formula I inhibit thioglycolate induced neutrophil migration when administered p.o. at a dose of from 0.001-50 mg/kg either at the time of thioglycolate injection or 3 hours before.

### ii) Allergic Contact Dermatitis (ACD)

Groups of 8 female NMRI mice are sensitized on the shaved abdomen with 50 µl of oxazolone (Sigma, 2% in acetone) and challenged with 10 µl of 0.2 or 2.0% oxazolone on the inner surface of the right ear 7 days later. The low concentration of oxazolone for induction of the elicitation phase is used for testing compounds on systemic activity whereas the high concentration is applied for systemic testing. The unchallenged left ears serve as normal controls and dermatitis is evaluated from the individual differences in pinnal weight, which is taken as a measure of increase in inflammatory swelling 24 h after the challenge. Dermatitis is evaluated in test- and for comparison in control groups. The test groups are treated with the test compounds either orally (twice, 2 h and immediately before challenge), subcutaneously (immediately before challenge) or topically (30 min after challenge at the site of elicitation of the ACD); the controls are treated similarly with the vehicles alone. For oral and subcutaneous administration the compounds are administered in an oil in water emulsion, for topical administration the compounds are prepared in a mixture of ethanol, acetone and dimethylacetamide. The data of the test- and the vehicle-treated control groups are statistically analysed by ANOVA followed by Dunnet T-test (normal distribution or data) or by H and U-test, respectively. When administered p.o. at a dose of from 0.1 to 10 mg/kg, compounds of formula I inhibit the elicitation phase of allergic contact dermatitis. Compound of Example 3 has an inhibiting effect in this assay of 40% when administered p.o. at a dose of 2 x 3 mg/kg, or of 50% when applied topically at a dose of 10 mM.

The compounds of formula I are, therefore, useful in the treatment and/or prevention of diseases or disorders mediated by LFA-1/ICAM-1, -ICAM-2 or -ICAM-3 interactions e.g. ischemia/reperfusion injury e.g. myocardial infarction, stroke, gut ischemia, renal failure or hemorrhage shock, acute or chronic rejection of organ or tissue allo- or xenografts, cancer, infection diseases such as septic shock, adult respiratory distress syndrome, or traumatic shock. The compounds of formula I are also useful in the treatment and/or prevention of acute or chronic inflammatory diseases or disorders or autoimmune diseases e.g. rheumatoid arthritis, osteoarthritis, systemic lupus erythematosus, hashimoto's thyroidis, multiple sclerosis, myasthenia gravis, diabetes type I and uveitis, asthma, inflammatory lung injury, inflammatory liver injury, inflammatory glomerular injury, cutaneous manifestations of immunologically-mediated disorders or illnesses, inflammatory and hyperproliferative skin diseases (such as psoriasis, atopic dermatitis, alopecia aerata, allergic contact dermatitis, irritant contact dermatitis and further eczematous dermatitises, seborrhoeic dermatitis, lichen planus, pemphigus, bullous pemphigoid, epidermolysis bullosa, urticaria, angioedemas, vasculitides, erythema multiforme, cutaneous eosinophilias, lupus erythematosus, acne, granuloma annulare, pyoderma gangrenosum, sun bums or toxic epidermal necrolysis), inflammatory bowel disease, Crohn's disease, ulcerative colitis, ophthalmic inflammatory diseases or immune-mediated conditions of the eye, such as autoimmune diseases, e.g. keratoplasty and chronic keratitis, allergic conditions, e.g. vernal conjunctivitis, inflammatory conditions and comeal transplants. For the above uses the required dosage will of course vary depending on the mode of administration, the particular condition to be treated and the effect desired. In general, satisfactory results are indicated to be obtained systemically at daily dosages of from about 0.1 to about 100 mg/kg body weight. An indicated daily dosage in the larger mammal, e.g. humans, is in the range from about 0.5 mg to about 500 mg, conveniently administered, for example, in divided doses up to four times a day or in retard form.

For topical use satisfactory results are obtained with local administration of a 1-3 % concentration of active substance several times daily, e.g. 2 to 5 times daily.

The compounds of formula 1 may be administered systemically or topically, by any conventional route, in particular enterally, e.g. orally, e.g. in the form of tablets or capsules, topically, e.g. in the form of lotions, gels, ointments or creams, or in a nasal or a suppository form. Percutaneous administration via patches or other delivery systems may also be a possible route for prevention or treatment of above diseases.

Pharmaceutical compositions comprising a compound of formula I in free form or in pharmaceutically acceptable salt form in association with at least one pharmaceutical acceptable carrier or diluent may be manufactured in conventional manner by mixing with a pharmaceutically acceptable carrier or diluent. Unit dosage forms for oral administration contain, for example, from about 0.1 mg to about 125 mg of active substance.

Topical administration is e.g. to the skin. A further form of topical administration is to the eye.

The compounds of formula I may be administered in free form or in pharmaceutically acceptable salt form e.g. as indicated above. Such salts may be prepared in conventional manner and exhibit the same order of activity as the free compounds.

In accordance with the foregoing the present invention further provides:
1.1 The use of a compound of formula 1 or a pharmaceutically acceptable salt thereof for the preparation of a medicament for preventing or treating disorders or diseases mediated by LFA-1/ICAM-1, -ICAM-2 or -ICAM-3 interactions, e.g. such as indicated above
1.2 The use of a compound of formula I or a pharmaceutically acceptable salt thereof for the preparation of a medicament for preventing or treating acute or chronic inflammatory diseases or disorders or autoimmune diseases, e.g. as indicated above, e.g. psoriasis or other inflammatory skin diseases,
2. A compound of formula 1, in free form or in a pharmaceutically acceptable salt form for use as a pharmaceutical, e.g. in any of the methods as indicated under 1.1 and 1.2 above.
3. A pharmaceutical composition, e.g. for use in any of the methods as in 1.1 and 1.2 above comprising a compound of formula 1 in free form or pharmaceutically acceptable salt form in association with a pharmaceutically acceptable diluent or carrier therefor.
4. A compound of formula I or a pharmaceutically acceptable salt thereof for use in the preparation of a pharmaceutical composition for use in any of the method as in 1.1 and 1.2 above.
   The compounds of formula I may be administered as the sole active ingredient or together with other drugs in immunomodulating regimens or other anti-inflammatory agents for the treatment or prevention of allo- or xenograft acute or chronic rejection or inflammatory or autoimmune disorders. For example, they may be used in combination with cyclosporins, rapamycins or ascomycins, or their immunosuppressive analogs, e.g. cyclosporin A, cyclosporin G, FK-506, ABT-281, ASM 981, rapamycin, 40-O-(2-hydroxy)ethyl-rapamycin etc.; corticosteroids; cyclophosphamide; azathioprene; methotrexate; FTY 720; leflunomide; mizoribine; mycophenolic acid; mycophenolate mofetil; 15-deoxyspergualine; immunosuppressive monoclonal antibodies, e.g., monoclonal antibodies to leukocyte receptors, e.g., MHC, CD2, CD3, CD4, CD7, CD25, CD28, B7, CD40, CD45, or CD58 or their ligands; or other immunomodulatory compounds, e.g. CTLA4Ig, or other adhesion molecule inhibitors, e.g. mAbs or low molecular weight inhibitors including Selectin antagonists and VLA-4 antagonists.
   Where the compounds of formula I are administered in conjunction with other immunosuppressive / immunomodulatory or anti-inflammatory therapy, e.g. for preventing or treating acute or chronic rejection or inflammatory or autoimmune disorders as hereinabove specified, dosages of the co-administered immunosuppressant, immunomodulatory or anti-inflammatory compound will of course vary depending on the type of co-drug employed, e.g. whether it is a steroid or a cyclosporin, on the specific drug employed, on the condition being treated and so forth. In accordance with the foregoing the present invention provides in a yet further aspect:
5. The use as defined above comprising co-administration, e.g. concomitantly or in sequence, of a therapeutically effective amount of a compound of formula I in free form or in pharmaceutically acceptable salt form, and a second drug substance, said second drug substance being an immunosuppressant, immunomodulatory or anti-inflammatory drug, e.g. as indicated above.
6. A therapeutic combination, e.g. a kit, for use in any method as defined under 1.1 or 1.2 above, comprising a compound of formula 1, in free form or in pharmaceutically acceptable salt form, to be used concomitantly or in sequence with at least one pharmaceutical composition comprising an immunosuppressant, immunomodulatory or anti-inflammatory drug. The kit may comprise instructions for its administration.

A preferred compound according to the invention is the compound of Example 3.

## Claims

1. A compound of formula I 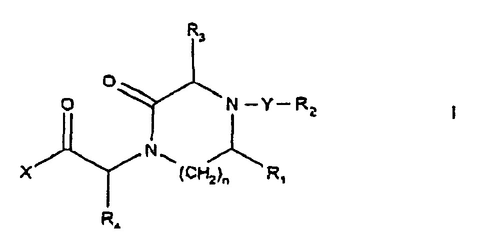
wherein
n is 1, 2 or 3,
R₁ is H, C₁₋₄alkyl; aryl; or aryl-C₁₋₄alkyl,
Y is C₁₋₄alkylene; -CO-C₁₋₄alkylene: -CO-C₂₋₅alkenylene; -CO-NH-; -CO-C₁₋₃alkylene-NH-; or -CO-O-,
R₂ Is phenyl, naphthyl, dihydro- or tetrahydro-naphthyl, biphenylyl, pyridyl, quinolyl, isoquinolyl, dihydro-, tetrahydro-quinolyl or -isoquinolyl, benzo-thienyl, indolyl or pyridyl-phenyt, each being optionally substituted by CF₃, halogen, OH, C₁-₄alkoxy, amino, mono- or di-C₁₋₄alkyl substituted amino, phenyl, benzyl or C₁₋₄alkyl optionally substituted by amino,
R₃ is the side chain present on the Cα of an α-amino acid,
R₄ is biphenylyl; or benzyl, hydroxy-benzyl, α- or β-naphthyl-methyl, 5,6,7,8-tetrahydro-β-naphthyl-methyl or indolyl-methyl, each being optionally substituted on the ring by CF₃, halogen, OH, C₁₋₄alkoxy, amino, mono- or di-C₁₋₄alkyl substituted amino, phenyl, benzyl or C₁₋₄alkyl optionally substituted by amino, and
X is -CN; -NR₅R₆; or -O-R₈ wherein R₅ is H, C₁₋₆alkyl, aryl or aryl-C₁₋₄alkyl, R₆ is H or C₁₋₆alkyl and R₈ is H, C₁₋₄alkyl, aryl or aryl-C₁₋₄alkyl,
in free form or in salt form.

2. A compound according to claim 1 wherein n is 2.

3. A compound according to claim 1 wherein R₂ is naphthyl; quinolyl; tetrahydro-quinolyl; tetrahydro-1-methyl-quinolyl; or quinolyl substituted by OH, OCH₃ or phenyl.

4. A compound according to claim 1 wherein R₄ is α- or β-naphthyl-methyl.

5. A compound according to claim 1, which is (R)-2-[(3S,5R)-5-Methyl-2-oxo-3-phenyl-4-(quinolin-6-yl-acetyl)-[1,4]diazepan-1-yl]-3-(naphthaien-1-yl)-propionamide in free form or in salt form.

6. A process for the preparation of a compound of formula I according to claim 1, which process comprises appropriately substituting a corresponding compound of formula II 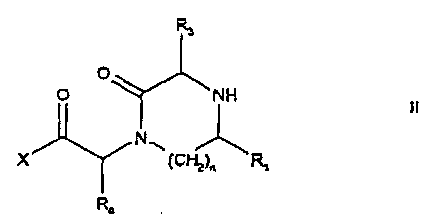
wherein R₁, R₃, R₄, X and n are as defined in claim 1,
and, where required, converting the resulting compound of formula I obtained in free form into a salt form or vice versa.

7. A compound of formula I according to claim 1, in free form or in a pharmaceutically acceptable salt form, for use as a pharmaceutical.

8. A pharmaceutical composition comprising a compound of formula I according to claim 1 in free form or pharmaceutically acceptable salt form in association with a pharmaceutically acceptable diluent or carrier therefor.

9. A pharmaceutical composition according to claim 8 for use concomitantly or in sequence with a second drug substance, said second drug substance being an immunosuppressant, immunomodulatory or anti-inflammatory drug.

10. Use of a compound of formula I according to claim 1 or a pharmaceutical acceptable salt thereof for the preparation of a medicament for preventing or treating disorders or diseases mediated by LFA-1/ICAM-1, -ICAM-2 or -ICAM-3 interactions.

## Patentansprüche

1. Verbindung der Formel I 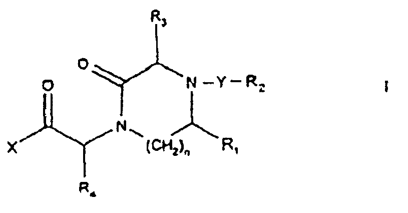
worin
n für 1, 2 oder 3 steht,
R₁ für H, C₁-C₄ Alkyl, Aryl oder Aryl-C₁-C₄-alkyl steht,
Y für C₁-C₄ Alkylen, -CO-C₁-C₄ Alkylen, -CO-C₂-C₅ Alkenylen, -CO-NH-, -CO-C₁-C₃ Alkylen-NH- oder -CO-O- steht,
R₂ steht für Phenyl, Naphthyl, Dihydro- oder Tetrahydronaphthyl, Biphenylyl, Pyridyl, Chinolyl, Isochinolyl, Dihydro-, Tetrahydro-chinolyl oder -isochinolyl, Benzothienyl, Indolyl oder Pyridylphenyl, das jeweils optional substituiert ist mit CF₃, Halogen, OH, C₁-C₄ Alkoxy, Amino, Mono- oder Di-C₁-C₄-alkyl substituiertem Amino, Phenyl, Benzyl oder C₁-C₄ Alkyl, das optional durch Amino substituiert ist,
R₃ für die Seitenkette steht, die auf dem Cα einer α-Aminosäure vorhanden ist,
R₄ steht für Biphenylyl oder Benzyl, Hydroxybenzyl, α- oder β-Naphthylmethyl, 5,6,7,8-Tetrahydro-β-naphthylmethyl oder Indotylmethyl, das jeweils am Ring optional substituiert ist durch CF₃, Halogen, OH, C₁-C₄ Alkoxy, Amino, Mono- oder Di-C₁-C₄-alkyl substituiertem Amino, Phenyl, Benzyl oder C₁-C₄ Alkyl, das wahlweise durch Amino substituiert ist, und
X für -CN, -NR₅R₆ oder -O-R₈ steht, worin R₅ für H, C₁-C₆ Alkyl, Aryl oder Aryl-C₁-C₄-alkyl steht, R₆ für H oder C₁-C₆ Alkyl steht und R₈ für H, C₁-C₄ Alkyl, Aryl oder Aryl-C₁-C₄-alkyl steht,
in freier Form oder Salzform.

2. Verbindung nach Anspruch 1, worin n für 2 steht.

3. Verbindung nach Anspruch 1, worin R₂ für Naphthyl, Chinolyl, Tetrahydrochinolyl, Tetrahydro-1-methylchinolyl oder Chinolyl steht, das durch OH, OCH₃ oder Phenyl substituiert ist.

4. Verbindung nach Anspruch 1, worin R₄ für α- oder β-Naphthylmethyl steht.

5. Verbindung nach Anspruch 1, die (R)-2-[(3S,5R)-5-Methyl-2-oxo-3-phenyl-4-(chinolin-6-yl-acetyl)-[1,4]diazepan-1-yl]-3-(naphthalin-1-yl)propionamid in freier Form oder in Salzform ist.

6. Verfahren zur Herstellung einer Verbindung der Formel 1 nach Anspruch 1, wobei das Verfahren umfasst eine geeignete Substitution einer entsprechenden Verbindung der Formel II 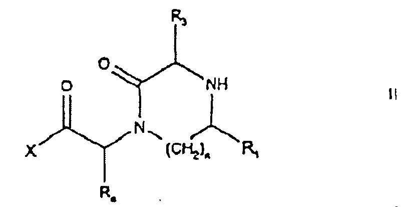
worin R₁, R₃, R₄, X und n wie in Anspruch 1 definiert sind,
und erforderlichenfalls eine Umwandlung der entstehenden Verbindung der Formel I, die als freie Form erhalten wird, in eine Salzform oder umgekehrt.

7. Verbindung der Formel 1 nach Anspruch 1 in freier Form oder in einer pharmazeutisch annehmbaren Salzform zur Verwendung als Pharmazeutikum.

8. Pharmazeutische Zusammensetzung, die eine Verbindung der Formel I nach Anspruch 1 in freier Form oder pharmazeutisch annehmbarer Salzform zusammen mit einem pharmazeutisch annehmbaren Verdünnungsmittel oder Träger hierfür enthält.

9. Pharmazeutische Zusammensetzung nach Anspruch 8 zur gleichzeitigen oder aufeinanderfolgenden Verwendung mit einer zweiten Arzneimittelsubstanz, wobei die zweite Arzneimittelsubstanz ein immunsuppressives, immunmodulatorisches oder antientzündliches Arzneimittel ist.

10. Verwendung einer Verbindung der Formel 1 nach Anspruch 1 oder eines pharmazeutisch annehmbaren Salzes hiervon zur Herstellung eines Arzneimittels zur Prävention oder Behandlung von Störungen oder Erkrankungen, die durch Wechselwirkungen von LFA-1 mit ICAM-1, ICAM 2 oder ICAM 3 vermittelt werden.

## Revendications

1. Composé de formule I 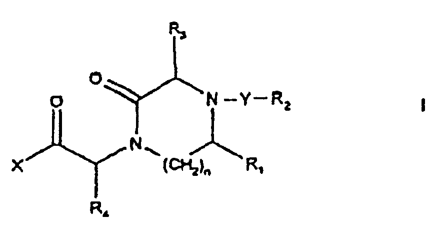
dans laquelle
n vaut 1, 2 ou 3,
R₁ représente H, un groupe alkyle en C₁₋₄, aryle ou aryl-(alkyle en C₁₋₄),
Y est un groupe alkylène en C₁₋₄, -CO-(alkylène en C₁₋₄), -CO-(alcénylène en C₂₋₅), -CO-NH-, -CO-(alkylène en C₁₋₃)-NH-, ou -CO-O-,
R₂ représente un groupe phényle, naphtyle, dihydro- ou tétrahydronaphtyle, biphénylyle, pyridyle, quinoléyle, isoquinoléyle, dihydro-, tétrahydro-quinoléyle ou -isoquinoléyle, benzothiényle, indolyle ou pyridylphényle, chacun étant éventuellement substitué par CF₃, un atome d'halogène, OH, un groupe alcoxy en C₁₋₄, amino, amino substitué par un mono- ou un di-(alkyle en C₁₋₄), phényle, benzyle ou alkyle en C₁₋₄ éventuellement substitué par un groupe amino,
R₃ est la chaîne latérale présente sur le C_{α} d'un acide α-aminé,
R₄ est un groupe biphényle ou benzyle, hydroxybenzyle, α- ou β-naphtylméthyle, 5,6,7,8-tétrahydro-β-naphtylméthyle ou indolylméthyle, chacun étant éventuellement substitué sur le noyau par CF₃, un atome d'halogène, OH, un groupe alcoxy en C₁₋₄, amino, amino substitué par un mono- ou un di-(alkyle en C₁₋₄), phényle, benzyle ou alkyle en C₁₋₄ éventuellement substitué par un groupe amino, et
X représente -CN, -NR₅R₆, ou -O-R₈, où R₅ représente H, un groupe alkyle en C₁₋₆, aryle ou aryl-(alkyle en C₁₋₄), R₆ représente H ou un groupe alkyle en C₁₋₆ et R₈ représente H, un groupe alkyle en C₁₋₄, aryle ou aryl-(alkyle en C₁₋₄),
sous forme libre ou sous forme de sel.

2. Composé selon la revendication 1, dans lequel n vaut 2.

3. Composé selon la revendication 1, dans lequel R₂ est un groupe naphtyle, quinoléyle, tétrahydroquinoléyle, tétrahydro-1-méthylquinoléyle ou quinoléyle substitué par un groupe OH, OCH₃ ou phényle.

4. Composé selon la revendication 1, dans lequel R₄ est un α- ou β-naphtylméthyle.

5. Composé selon la revendication 1, qui est le (R)-2-[(3S,5R)-5-méthyl-2-oxo-3-phényl-4-(quinoléin-6-yl-acétyl)-[1,4]-diazépan-1-yl)-3-(naphtalén-1-yl)-propionamide sous forme libre ou sous forme de sel.

6. Procédé pour la préparation d'un composé de formule I selon la revendication 1, lequel procédé comprend la substitution de manière appropriée d'un composé de formule II correspondant 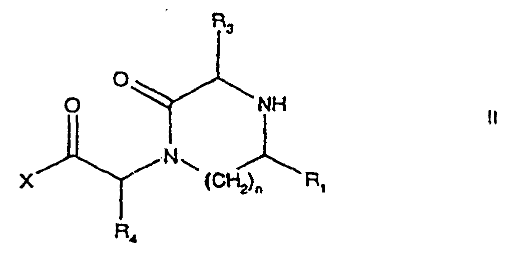
dans laquelle R₁, R₃, R₄, X et n sont tels que définis dans la revendication 1, et si nécessaire, la conversion du composé de formule I résultant, obtenu sous forme libre, en un composé sous forme de sel ou vice versa.

7. Composé de formule I selon la revendication 1, sous forme libre ou sous forme d'un sel pharmaceutiquement acceptable, à utiliser en tant qu'agent pharmaceutique.

8. Composition pharmaceutique comprenant un composé de formule I selon la revendication 1, sous forme libre ou sous forme d'un sel pharmaceutiquement acceptable, en association avec un diluant ou véhicule pharmaceutiquement acceptable pour ce composé.

9. Composition pharmaceutique selon la revendication 8, à utiliser de manière concomitante ou successivement, avec une deuxième substance médicamenteuse, ladite deuxième substance médicamenteuse étant un médicament immunosupresseur, immunomodulateur ou anti-inflammatoire.

10. Utilisation d'un composé de formule I selon la revendication 1 ou d'un de ses sels pharmaceutiquement acceptables pour la préparation d'un médicament destiné à la prévention ou au traitement de troubles ou de maladies à médiation par les interactions LFA-1/ICAR4-1, -ICAM-2 ou -ICAM-3.
